# EUROPÄISCHE PATENTSCHRIFT

(11) **EP 0 390 896 B1**
(45) Veröffentlichungstag und Bekanntmachung des Hinweises auf die Patenterteilung: **28.12.1994**
(21) Anmeldenummer: 89911012.6
(22) Anmeldetag: 09.10.1989
(51) Int. Cl.: A61B 3/024, A61B 3/12

(54) **VORRICHTUNG ZUR GESICHTSFELDPRÜFUNG**
DEVICE FOR EXAMINING THE FIELD OF VISION
DISPOSITIF DE CONTROLE DU CHAMP VISUEL

(30) Priorität: 08.10.1988 DE 3834327; 07.07.1989 DE 3922471
(43) Veröffentlichungstag der Anmeldung: 10.10.1990
(73) Patentinhaber: G. RODENSTOCK INSTRUMENTE GMBH, 85521 Ottobrunn (DE)
(72) Erfinder: KLINGBEIL, Ulrich, D-8000 München 40 (DE); PLESCH, Andreas, D-8000 München 40 (DE); RAPPL, Wolfgang, D-8000 München 2 (DE)
(74) Vertreter: Münich, Wilhelm, Dr.
(86) Internationale Anmeldenummer: DE8900643
(87) Internationale Veröffentlichungsnummer: WO9003759

(56) Entgegenhaltungen:
- WO-A-88/03396
- GB-A- 2 114 406
- US-A- 3 827 789
- US-A- 4 012 128
- US-A- 4 260 227
- US-A- 4 558 933

## Beschreibung

### Technisches Gebiet

Die Erfindung bezieht sich auf eine Vorrichtung zur Gesichtsfeldprüfung gemäß dem Oberbegriff des Patentanspruchs 1.

### Stand der Technik

Die gegenwärtig auf dem Markt erhältlichen Vorrichtungen zur Gesichtsfeldprüfung weisen eine Halbkugelschale auf in der nacheinander über das Gesichtsfeld verteilte Lichtpunkte einstellbarer Helligkeit, die auch als Stimuli bezeichnet werden, aufleuchten. Diese Lichtpunkte einstellbarer Helligkeit werden dabei durch in der Halbkugelschale angebrachte Leuchtdioden oder ein Projektionssystem erzeugt. Die untersuchte Person, die eine sog. Fixationsmarke fixiert, gibt bspw. durch das Drücken einer Taste die Antwort "gesehen" bzw. "nicht gesehen". Eine Steuerung registriert die gegebene Antwort in Zuordnung zum Ort und zur Helligkeit des aufgeleuchteten Lichtpunktes. Das Ergebnis, das i.d. R. graphisch dargestellt wird, gibt dann eine "Karte" von den absoluten oder relativen Gesichtsfeld-Defekten.

Diese bekannten Vorrichtungen haben jedoch eine Reihe von Nachteilen:
Die Helligkeit eines Lichtpunktes kann zwar eingestellt werden. Der eingestellte Helligkeitswert steht aber in keiner strengen Korrelation zu der tatsächlich auf den Augenhintergrund gelangenden Lichtintensität, da diese nicht nur von dem Helligkeitswert des aufleuchtenden Lichtpunktes, sondern auch noch von der Pupillengröße etc. abhängt.

Weiterhin ist bei den bekannten Vorrichtungen sicherzustellen, daß der Patient die Fixationsmarke fixiert und "nicht in der Halbkugelschale umherblickt", da ansonsten die Antwort "gesehen" nicht aussagekräftig für das Fehlen von Gesichtsfeld-Defekten ist.

Insbesondere besteht bei den bekannten Vorrichtungen keine direkte Korrelationsmöglichkeit zwischen dem Ort eines aufleuchtenden Lichtpunktes und evtl. auf dem Augenhintergrund erkennbaren markanten bzw. pathologischen Strukturen, da keine direkte Beobachtung des Augenhintergrundes für die Untersuchungsperson während der Gesichtsfeldprüfung möglich ist.

Vor allem aber sind die vorgenannten bekannten Vorrichtungen zur Gesichtsfeldprüfung auf die "Mithilfe" der untersuchten Person angewiesen, d.h. sie erlauben keine sog. objektive Visusprüfung. Bei der sog. objektiven Visusbestimmung soll der Visus, also das Sehvermögen eines Probanten ermittelt werden, ohne daß eine Kooperation des Untersuchten erforderlich ist. Typische Anwendungsfälle für eine objektive Visusbestimmung wären die Überführung von Simulanten bei Gerichtsgutachten oder die Visusuntersuchung von Kleinkindern.

In der wissenschaftlichen Literatur ist vorgeschlagen worden, zur objektiven Visusbestimmung einen sog. optokinetischen Nystagmus auszulösen. Hierunter wird verstanden, daß der untersuchten Person ein bewegtes Muster bzw. eine Marke als Stimulus dargeboten wird. Wird dieses Muster wahrgenommen, d.h. sind die Strukturen im Muster groß genug, um vom Auge aufgelöst zu werden, so führt die Stimulus-Bewegung zu charakteristischen, unwillkürlichen Folgebewegungen des Auges, dem sog. optokinetischen Nystagmus.

Es ist jedoch bislang keine Vorrichtung bekannt geworden, mit der in der Praxis eine objektive Visusbestimmung möglich wäre.

Der Grund hierfür ist, daß es gerade Simulanten immer wieder gelingt, bewußt an dem Stimulus vorbeizuschauen, und damit die Untersuchungsperson zu täuschen.

Weiterhin ist eine Vorrichtung zur Gesichtsfeldprüfung vorgeschlagen worden, bei der ein sog. Scanning-Laser-Ophthalmoskop verwendet wird, bei dem die Intensität des Abtast-Lichtstrahls zur Erzeugung der Marken bzw. Stimuli moduliert wird. Derartige Vorrichtungen sind bspw. in dem Artikel "Scanning Laser Ophthalmoscopy", Ophthalmology, Vol. 89, Nr. 7 Juli 1982, Seite 852 - 857 oder in dem Artikel "Reading With a Macular Scotoma", in "Investigative Ophthalmology and Visual Science", Juli 1986, Vol. 27, Seite 1137 - 1147 beschrieben, auf die im übrigen zur Erläuterung aller hier nicht näher beschriebenen Einzelheiten ausdrücklich Bezug genommen wird.

Von der in diesen Artikeln beschriebenen Vorrichtung ist im übrigen bei der Formulierung des Oberbegriffs des Patentanspruchs 1 ausgegangen worden. Eine weitere Vorrichtung zur Gesichtsfeldprüfung der genannten Art ist aus WO-A-88/03396 bekannt.

Die Verwendung eines sog. Scanning-Laser-Ophthalmoskops zur Gesichtsfeldprüfung hat den Vorteil, daß die Gesichtsfeldprüfung unter visueller Beobachtung durch die Untersuchungsperson stattfinden kann, da die zur Erzeugung eines Bildes des Augenhintergrundes erforderliche Lichtmenge so gering ist, daß sie als "Untergrund-Helligkeit" praktisch nicht stört. Darüberhinaus haben Scanning-Laser-Ophthalmoskope insbesondere dann, wenn sie die in der US-PS 4 213 678 beschriebene "Pupillenteilung" zwischen der Eintrittspupille für den Beleuchtungslichtstrahl und der Austrittspupille für das am Augenhintergrund reflektierte Licht verwenden, den Vorteil, daß der Beleuchtungslichtstrahl einen kleinen, zentralen Teil der Augenpupille passiert, so daß die auf den Augenhintergrund gelangene Lichtquelle nicht von der Größe der Pupille abhängt.

Ferner können die Stimuli nicht nur Lichtpunkte, sondern auch kompliziertere Muster, wie beispielsweise Landold-Ringe sein.

Bei den aus den vorstehend genannten Veröffentlichungen bekannten Vorrichtungen wird zur Steuerung der Intensität des Beleuchtungslichtstrahls ein akusto-optischer Modulator verwendet, der - je nach dem ob gerade nur ein Bild des Augenhintergrundes oder ein "Stimulus"-Muster erzeugt werden soll - einen mehr oder weniger großen Teil des Beleuchtungslichtstrahls passieren läßt.

Mit bekannten akusto-optischen Modulatoren kann die Intensität des Beleuchtungslichtstrahls etwa um den Faktor 100 variiert werden. Eine derartige Intensitäts-Variation ist jedoch bei einer Vorrichtung zur Gesichtsfeldprüfung nicht ausreichend, da es bspw. zum Ausloten von sog. relativen Gesichtsfeld-Defekten d.h. Gesichtsfeld-Stellen, an denen helle Lichtpunkte noch, dunkle Lichtpunkte aber nicht mehr wahrgenommen werden, erforderlich ist, die Lichtintensität physiologisch um etwa 40 bis 50 dB zu variieren.

Darüberhinaus ist es praktisch nicht möglich, ein Muster mit unterschiedlicher Helligkeit zu projizieren.

Auch diese bekannte Vorrichtung gemäß dem Oberbegriff des Anspruchs 1 erlaubt keine objektive Visusprüfung, sondern ist auf die Kooperation der untersuchten Person angewiesen.

### Darstellung der Erfindung

Der Erfindung liegt die Aufgabe zugrunde, eine Vorrichtung zur Gesichtsfeldprüfung gemäß dem Oberbegriff des Patentanspruchs 1 derart weiterzubilden, daß die Intensität des Beleuchtungslichtstrahls um wenigstens 40 dB oder mehr variiert werden kann. Eine weitere Aufgabe der Erfindung besteht darin, eine Vorrichtung zur objektiven Gesichtsfeldprüfung bzw. zur objektiven Visusbestimmung anzugeben, mit der insbesondere der Visus von Personen, die die Untersuchungsperson über ihre Sehfähigkeit täuschen wollen, objektiv ermittelbar ist.

Eine erfindungsgemäße Lösung der ersten Aufgabe ist im Anspruch 1 gekennzeichnet: Erfindungsgemäß schaltet die Steuereinheit zum Einstellen eines bestimmten Helligkeitswertes der Marken (Muster) bzw. Stimuli den Beleuchtungslichtstrahl innerhalb der Zeitspanne, in der der Beleuchtungslichtstrahl einen Abtastpunkt abtastet, wenigstens einmal von einem ersten Intensitätswert auf einen zweiten Intensitätswert um, Dieser zweite Intensitätswert kann insbesondere gemäß Anspruch 7 den Wert Null haben, d.h. die Steuereinheit schaltet den Beleuchtungslichtstrahl wenigstens einmal für einen bestimmten Bruchteil dieser Zeitspanne ab.

Erfindungsgemäß ist damit die Intensität des Beleuchtungslichtstrahles während der Beleuchtung eines "Abtastpunktes" nicht konstant, wie dies bei den bekannten Vorrichtungen gemäß dem Oberbegriff des Patentanspruchs 1 der Fall ist. Da andererseits die Zeitdauer der Beleuchtung eines Abtastpunktes - je nach Zahl der aufgenommenen "Pixel" - ca. 100 ns beträgt, wird das Umschalten des Beleuchtungslichtstrahles, das damit zwangsläufig zu "Pulsdauern für die einzelnen Intensiätswerte von weniger als 100 ns führt, von der untersuchten Person nicht wahrgenommen. Die Netzhaut der untersuchten Person registriert vielmehr einen mittleren Helligkeitswert, der den zeitlichen Integral über die auftreffende Luftintensität entspricht.

Durch diese erfindungsgemäße Maßnahme kann die Helligkeit der von Beleuchtungslichtstrahl geschriebenen Marken in einem wesentlich größeren Rahmen als bei den bekannten Vorrichtungen variiert werden, bei denen der Transmissionsfaktor des akusto-optischen Modulators zwischen einem Minimal- und einem Maximal-Wert variiert wird, wobei während eines "Abtastpunktes" sich der Transmissionsfaktor des akusto-optischen Modulators nicht ändert. Insbesondere ist es damit auch möglich, Marken zu erzeugen, die eine Reihe von "Abtastpunkten umfassen" und bei der die Helligkeit so variiert, daß die Variation vom menschlichen Auge aufgelöst wird. Dies kann beispielweise bei der Untersuchung sog. rezeptiver Felder von Vorteil sein.

Weiterhin ist erfindungsgemäß erkannt worden, daß es unter Verwendung einer Vorrichtung gemäß Anspruch 1 möglich ist, objektiv den Visus einer untersuchten Person unter gleichzeitiger Fixationskontrolle zu bestimmen.

Erfindungsgemäß werden durch Umschalten des Lichtstrahls der Beleuchtungslichtquelle derartiger Scanning-Ophthalmoskope zwischen wenigstens zwei Helligkeitsstufen Muster auf dem Augenhintergrund projiziert, die einen optokinetischen Nystagmus auslösen, und gleichzeitig unwillkürliche Augenbewegung aufgrund der Projektion der Muster zu verfolgt.

Dabei ist es von besonderem Vorteil, daß die "normale Helligkeit" des Abtast-Lichtstrahls bei einem Scanning-Ophthalmoskop so gering sein kann, daß sie unter der "Reizschwelle" bleiben kann. Damit ist gemeint, daß die untersuchte Person die projizierten Muster nicht auf einem "sehr hohem Helligkeitsniveau" wahrnimmt, wie es bspw. erforderlich wäre, wenn während der Projektion der Muster der Augenhintergrund mit einer herkömmlichen Funduskamera mit einer flächigen Beleutungslichtquelle beobachtet werden würde.

Weiterhin ist von Vorteil, daß gemäß Anspruch 4 leicht beliebige Muster durch den modulierten Beleuchtungslichtstrahl erzeugt werden können, wobei es ohne Schwierigkeiten möglich ist, die Muster über vorgebbare Bereiche des Augenhintergrunds zu bewegen.

Die Reaktion der untersuchten Person kann bspw. vom Untersucher visuell an einem Monitor kontrolliert werden, auf dem ein Bild des Augenhintergrunds dargestellt ist. Besonders vorteilhaft ist es jedoch, daß durch die erfindungsgemäße Ausbildung, bei der "gleichzeitig" mit der Projektion der Muster der Augenhintergrund aufgenommen wird, die Steuer- und Auswerteeinheit Bewegungen des Auges erfaßt und mit dem Ort auf dem Augenhintergrund korrelliert, auf dem das oder die Muster projiziert werden. Zusätzlich ist gemäß Anspruch 6 eine Kompensation willkürlicher Augenbewegungen über ein sog. "Fundustracking" möglich, wie es prinzipiell für den Bereich der Ophthalmologie bekannt ist.

Da die erfindungsgemäße Vorrichtung selbsttätig erkennt, ob die dargebotene Struktur von der untersuchten Person gesehen wird, ist es darüber hinaus auch möglich, durch Variation der Helligkeit des Lichtstrahls in mehr als zwei Stufen oder durch eine kontinuierliche Variation eine automatische Visus-Schwellwertmessung durchzuführen, bei dem die Struktur bei Erkennen so lange sukzessive in ihrer Helligkeit verringert, bzw. die Strukturgröße sukzessive verkleinert wird, bis der sog. optokinetische Nystagmus nicht mehr ausgelöst wird (Anspruch 3). Selbstverständlich ist es aber auch möglich, die erfindungsgemäße Vorrichtung in bekannter Weise zu betreiben, so daß die untersuchte Person die Antwort "gesehen" bzw. "nicht gesehen" beispielsweise durch Drücken einer Taste gibt. Die Steuereinheit, die beispielsweise einen handelsüblichen Mikrocomputer aufweisen kann, speichert dann die Antwort der untersuchten Person in Zuordnung zum Ort, der Größe und der Helligkeit des jeweiligen Musters bzw. Marke.

Um die Störung der untersuchten Person durch das "normale Beleuchtungslicht" so gering wie möglich zu halten, ist es darüberhinaus auch möglich, bei einer erfindungsgemäßen Vorrichtung mit zwei Wellenlängen zu arbeiten, also bspw. die normale Fundusbeobachtung mit Infrarotlicht vorzunehmen, das von der untersuchten Person nicht wahrgenommen wird, und sichtbares Licht lediglich zur Projektion der Muster bzw. Marken einzublenden (Anspruch 12).

Selbstverständlich ist es möglich, daß die Steuereinheit zusätzlich die Intensität des Beleuchtungslichtstrahles variiert (Anspruch 8). Dies kann bspw. dann, wenn als Beleuchtungslichtquelle eine Leuchtdiode oder eine Laser-Diode verwendet wird, durch Variation der Betriebsspannung der Diode erfolgen.

Das Umschalten bzw. das Abschalten und anschließende Wieder-Anschalten kann selbstverständlich auf die unterschiedlichste Art und Weise, bspw. durch entsprechendes An- und Abschalten der Beleuchtungslichtquelle erfolgen (Anspruch 10).

Weiterhin ist es möglich, daß die Steuereinheit einen akusto-optischen Modulator aufweist, der im Beleuchtungs-Lichtweg angeordnet ist (Anspruch 9). Dieser akusto-optische Modulator kann dabei zum Einstellen des Intensitäts-Wertes der Beleuchtungslichtquelle dienen oder er wird hochfrequent derart angesteuert, daß er während der Bilderzeugung an den Stellen, an denen lediglich der Augenhintergrund abgebildet werden soll, "die meiste Zeit" den Lichtweg blockiert und damit nur einen kleinen Teil des Beleuchtungslichtstrahles durchläßt, während er bei einer Abtasteinrichtung, bei der Marken geschrieben werden sollen, einen größeren Bruchteil der Abtastzeit den Beleuchtungslichtweg öffnet und damit einen entsprechenden größeren Anteil des Beleuchtunglichtes hindurch treten läßt.

Ferner ist es möglich, daß gemäß Anspruch 11 eine weitere Lichtquelle vorgesehen ist, die eine Untergrundhelligkeit erzeugt, so daß die Gesichtsfeldprüfung "auf einem bestimmten Niveau der Umgebungshelligkeit" stattfindet. Ausdrücklich wird aber darauf hingewiesen, daß die gewünschte "Untergrundhelligkeit" auch durch den abtastenden Beleuchtungslichstrahl eingestellt werden kann, der ebenfalls "im Mittel" eine bestimmte "Untergrundhelligkeit" erzeugen kann.

Vorrichtungen zur Gesichtsfeldprüfung gemäß Patentanspruch 1 haben gegenüber anderen Vorrichtungen zur Gesichtsfeldprüfung, bei denen Leuchtdioden zur Erzeugung der Marken vorgesehen sind, den Vorteil, daß "Stimuli" an beliebigen Stellen im Gesichtsfeld mit frei vorgebbarer Form erzeugt werden können. Deshalb weist die Steuereinheit bevorzugt einen sog. (Bild-) Speicher auf, in dem die Position, Form und Helligkeit der Marken gespeichert sind, die nacheinander der untersuchten Person dargeboten werden sollen (Anspruch 13) und der gegebenenfalls die Antwort der untersuchten Person in Zuordnung zum Ort und der Größe bzw. Helligkeit der jeweiligen Marke (Anspruch 4 Anspruch 14) bzw. eventuell auftretende "unwillkürliche" Bewegungen des Auges speichert. Es versteht sich dabei von selbst, daß dabei bestimmte Abläufe in der Darbietung der einzelnen Marken eingehalten werden können, wie dies aus der Patentliteratur oder aus Geräten der Anmelderin der vorliegenden Anmeldung bekannt ist. Insbesondere kann zunächst eine Abfrage von bestimmten grob über das Gesichtsfeld verteilten Marken mit großer Helligkeit erfolgen; anschließend können dann erkannte Defekte näher "ausgelotet" werden.

Die erfindungsgemäße Vorrichtung hat dabei den besonderen Vorteil, daß die einzelnen Daten der Marken in Zuordnung zum aufgenommen Bild bzw. von markanten Fundusstellen gespeichert werden können: Hierzu wird gleichzeitig mit der Raster- bzw. Markenprojektion ein Bild des untersuchten Fundus aufgenommen und die Lage einer markanten Fundustextur analysiert. Dazu wird vor Beginn der Untersuchung eine solche Fundusstruktur (Template) ausgewählt. Bei allen nachfolgenden während jeder Rasterprojektion aufgenommenen Bildern wird untersucht, wie weit sich die markierte Struktur gegenüber der Lage der Struktur im ursprünglichen oder vorausgegangenen Bild verschoben hat. Dies kann automatisch über ein sog. Fundus-Tracking oder halbautomatisch über eine Markierung der markanten Struktur erfolgen. Die jeweils festgestellten Verschiebungen werden benutzt, um Bewegungen des Fundus während der Untersuchung zu kompensieren bzw. die Rasterlage nachträglich zu korrigieren (Ansprüche 14 bzw. 15).

Diese Nachführung ermöglicht erstmals eine lagegerechte Empfindlichkeitsuntersuchung des Gesichtsfeldes.

Die erfindungsgemäß vorgesehene digitale Speicherung des Ortes, der Größe und der Helligkeit der einzelnen Marken (Anspruch 16) wird durch die im Anspruch 17 angegebenen Merkmale weiter vorteilhaft ausgebildet: Demgemäß ist die Helligkeit der Marken beispielsweise als 8-Bit-Wert gespeichert. Da mit 8-Bit lediglich zweieinhalb Größenordnungen darstellbar sind, wird der gespeicherte 8-Bit-Wert erfindungsgemäß nicht linear einem Helligkeitswert zugeordnet Dies kann bspw. dadurch geschehen, daß der im Bildspeicher gespeicherte 8-Bit-Wert zunächst digital/analog gewandelt wird und der analog gewandelte Wert durch wenigstens einen logarithmischen Verstärker mit fester oder programmierbarer Verstärkung in einen Grauwert, d.h. einen Helligkeitswert umgesetzt wird.

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:

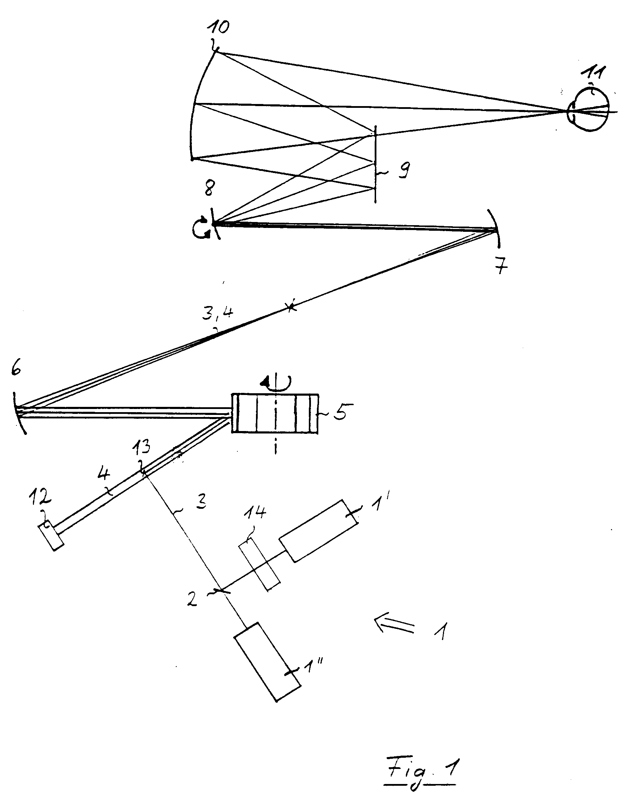

- Fig. 1: den Grundaufbau einer erfindungsgemäßen Vorrichtung,
- Fig. 2: das Prinzip der erfindungsgemäßen Helligkeits-Variation, und
- Fig. 3: die erfindungsgemäße Helligkeitsvariation mit zusätzlicher Intensitätsvariation.

### Darstellung eines Ausführungsbeispiels

Die erfindungsgemäße Vorrichtung kann bspw. mit einem Laser-Scanning-Ophthalmoskop realisiert werden, wie es in der WO 88/033 96 beschrieben ist. Diese Vorrichtung weist eine Beleuchtungs-Lichtquelle 1 auf, die bei dem gezeigten Ausführungsbeispiel aus zwei Lagern 1' und 1'' besteht, die mit unterschiedlichen Wellenlängen arbeiten und mittels eines Spiegels 2 alternativ oder gemeinsam einen Beleuchtungslichtstrahl 3 erzeugen. Bei dem gezeigten Ausführungsbeispiel "verlaufen" sowohl der Beleuchtungslichtstrahl 3 als auch der vom Augenhintergrund kommende Lichtstrahl 4 über die Ablenkeinrichtung, die im folgenden näher beschrieben wird.

Der Lichtstrahl 3 der Beleuchtungs-Lichtquelle 1 wird von einem Horizontal-Scanner, der bei dem gezeigten Ausführungsbeispiel ein sich drehender Polygonspiegel 5 ist, in Horizontalrichtung abgelenkt. Der nun in der Horizontalebene aufgefächerte Strahl durchläuft das Spiegelsystem 6 und 7, und trifft auf einen Vertikal-Scanner, der bei dem gezeigten Ausführungsbeispiel ein Schwing- bzw. Galvanometerspiegel 8 ist, auf. Nach dem Spiegel 8 hat das Strahlbündel einen "rechteckigen" Querschnitt. Nach Umlenkung an einem Planspiegel 9 wird er von einem Konkavspiegel 10 auf das zu untersuchende Auge 11 abgebildet. Der am Augenhintergrund reflektierte Lichtstrahl 4 durchläuft in umgekehrter Reihenfolge die genannten Elemente und wird nach dem Horizontal-Ablenkelement 5 von einer Detektoreinheit 12 nach vorheriger Trennung des Beleuchtungs- und des Beobachtungslichtwegs mittels eines Spiegels 13 nachgewiesen. Durch die Kombination von Spiegeln als abbildende Elemente ergeben sich eine Reihe von Vorteilen, wie geringe Abbildungsfehler, Reflexfreiheit, Achromazität sowie durch die Faltung des Strahlenganges ein geringer Platzbedarf. Dabei ist die Achromazität von besonderer Bedeutung, wenn die Beleuchtung gleichzeitig mit Laserlicht unterschiedlicher Wellenlänge, beispielsweise im Infrarotbereich und im sichtbaren Bereich erfolgt.

Der Teilerspiegel 13, der den Beleuchtungslichtweg 3 und den Beobachtungslichtweg 4 trennt, ist bei dem gezeigten Ausführungsbeispiel ein kleiner Spiegel, so daß die Austrittspupille des reflektierten Strahlengangs 4 die Eintrittspupille ringförmig umgibt. Selnstverständlich ist es aber auch möglich, andere Teilerspiegel 13 zu verwenden, die beispielsweise zu einer Überlagerung der Eintritts- und Austrittspupille führen.

Ferner kann im Beleuchtungslichtweg 3 ein akusto-optischer Modulator 14 vorgesehen sein, der das bspw. von dem Laser 1' emittierte Licht in dem einen Schalt-Zustand passieren läßt und in dem anderen Schalt-Zustand blockiert. Dabei erfolgt das An- und Abschalten des Modulators so schnell, daß während jeder Ausleuchtzeit eines Bildpunktes der Lichtweg wenigstens einmal blockiert ist. Der akustooptische Modulator 14 kann aber auch dazu verwendet werden, die Intensität des Laserstrahls "integral" einzustellen, wobei dann die Umschaltung zwischen den beiden Intensitätswerten beispielsweise durch geeignetes Ansteuern der des Laser erfolgt. Diese Ausbildung ist bei der "Geschwindigkeit" der gegenwärtig handelsüblichen Modulatoren bevorzugt. Die Ausleuchtzeit eines Bildpunktes hängt natürlich von der Zahl der erzeugten Bilder und von der Zahl der Bildpunkte pro Bild ab, bei der üblichen Videonorm beträgt sie typischerweise 100 ns.

Da damit notwendigerweise die Zeit, während der der Lichtweg blockiert ist, kleiner als 100 ns sein muß, nimmt das Auge den An- und Abschalt-Vorgang nicht wahr, sondern "sieht lediglich eine gemittelte integrierte Intensität".

Fig. 2 zeigt dies schematisch für den Fall, daß während der Beleuchtungszeit eines Bildpunktes der Beleuchtungslichtstrahl viermal ab- und entsprechend wieder angeschaltet wird, so daß sich insgesamt fünf Beleuchtungsperioden ergeben. Die tatsächlich vom Auge wahrgenommene Intensität hängt dann vom Verhältnis Anschaltzeit/Abschaltzeit ab.

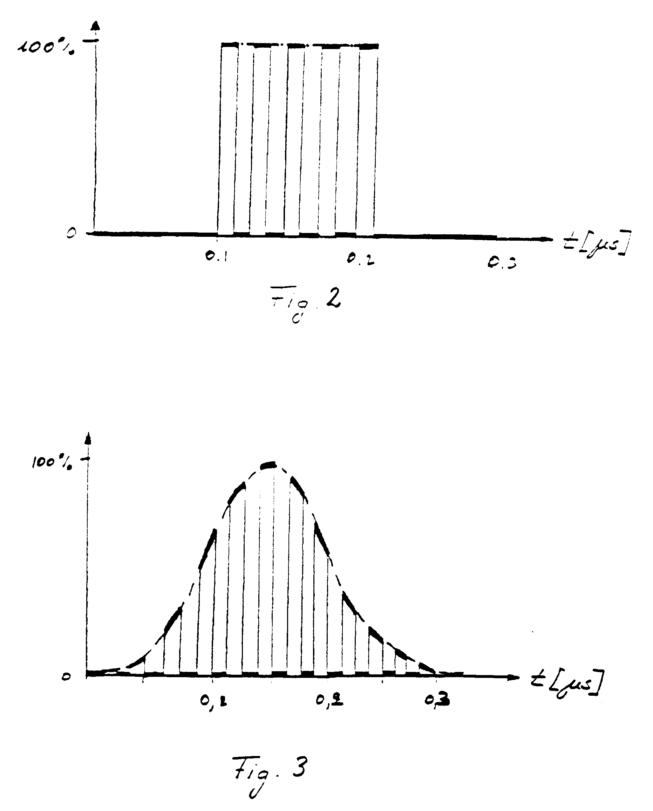

Fig. 3 zeigt schematisch den Fall, daß zusätzlich die Intensität des Laserstrahls beispielweise durch den akusto-optischen Modulator 14 oder entsprechendes Ansteuern der Laser 1' bzw. 1'' variiert wird.

Die Kombination von An- und Abschalten des Lichtstrahls in Verbindung mit einer Intensitätsvariation erlaubt eine Variation der vom Auge wahrgenommenen Helligkeit um wenigstens 50 dB.

Vorstehend ist die Erfindung anhand eines Ausführungsbeispieles ohne Beschränkung des allgemeinen Erfindungsgedankens beschrieben worden. So können als "Grundvorrichtungen" auch andere Vorrichtungen als beschrieben verwendet werden. Ausdrücklich wird ferner daraufhingewiesen, daß als Lichtquellen beliebige Lichtquellen, d.h. auch andere Lichtquellen als Laser verwendet werden können.

Ferner ist es möglich, eine zusätzliche Beleuchtungslichtquelle vorzusehen, die eine "Untergrundhelligkeit" erzeugt oder die Aufnahme eines Infrarot-Bildes gestattet. Da das Infrarot-Licht vom Auge nicht wahrgenommen wird, ist es damit möglich, zu überprüfen, ob sehr dunkle Lichtpunkte bei geringer Untergrundhelligkeit wahrgenommen werden können. Trotzdem gestattet die erfindungsgemäße Vorrichtung eine visuelle Beobachtung der Lage der projizierten Muster auf dem Augenhintergrund.

Weiterhin kann die erfindungsgemäße Vorrichtung zur Messung von evozierten Potentialen oder anderer elektrophysikalischer Daten verwendet werden. Auch können anstelle des akusto-optischen Modulators andere Maßnahmen zum An- und Abschalten des Beleuchtungslichtstrahles verwendet werden. Bspw. ist es möglich, die Lichtquelle an- und abzuschalten.

Die vorstehend nicht näher beschriebene Steuereinheit, die die Steuerung der Lichtpunkte sowie gegebenenfalls eine Nachführung bei Augenbewegungen vornimmt, kann in an sich bekannter Weise beispielsweise mit einem Mikrocomputer realisiert werden.

## Patentansprüche

1. Vorrichtung zur Gesichtsfeldprüfung, mit
(a) einer Beleuchtungslichtquelle (1), die einen Beleuchtungslichtstrahl (3) erzeugt,
(b) einer Detektoreinrichtung (12) mit nachgeschalteter Auswerte- und Synchronisiereinheit,
(c) einer Abtasteinrichtung, die strahlablenkende (5,8) und strahlabbildende Elemente (6,7,10) aufweist, über die der Beleuchtungslichtstrahl längs eines Lichtwegs auf einen vorbestimmten Bereich des Augenhintergrundes eines zu untersuchenden Auges (11) abgebildet wird, und die das vom Augenhintergrund reflektierte Licht (4) zu der Detektoreinrichtung führen, aus deren zeitsequentiellen Ausgangssignal die Auswerte- und Synchronisiereinheit punktweise ein Bild des beleuchteten Bereichs des Augenhintergrunds erzeugt, und
(d) einer Steuereinheit, die die Intensität des den Augenhintergrund abtastenden Beleuchtungslichtstrahls (3) derart steuert, daß auf einen vorbestimmbaren Bereich des Augenhintergrundes Marken bzw. Muster mit einer vorgebbaren Helligkeit projiziert werden, die die untersuchte Person wahrnimmt bzw. bei Gesichtsfeld-Defekten nicht wahrnimmt,
dadurch **gekennzeichnet**, daß die Steuereinheit derart ausgebildet ist, daß sie innerhalb der Zeitspanne, in der der Beleuchtungslichtstrahl einen vorbestimmten Bereich, auf den Marken bzw. Muster projiziert werden sollen, beleuchtet, den Beleuchtungslichtstrahl (3) zum Einstellen eines bestimmten Helligkeitswertes der Marken bzw. Muster wenigstens einmal für einen bestimmten Bruchteil dieser Zeitspanne von einem ersten Intensitätswert auf mindestens einen zweiten Intensitätswert umschaltet.

2. Vorrichtung nach Anspruch 1,
dadurch **gekennzeichnet**, daß die Steuereinheit zur objektiven Visusprüfung Bewegungen des Auges erfaßt und mit dem Ort des vorbestimmten Bereichs auf dem Augenhintergrund korrelliert, auf den das oder die Marken bzw. Muster projiziert werden.

3. Vorrichtung nach Anspruch 1 oder 2,
dadurch **gekennzeichnet**, daß die Steuereinheit die Musterhelligkeit und/oder -größe sowie gegebenenfalls deren Bewegungsgeschwindigkeit einstellbar verändert.

4. Vorrichtung nach Anspruch 3,
dadurch **gekennzeichnet**, daß die Steuereinheit die Antwort "gesehen" bzw. "nicht gesehen" der untersuchten Person in Zuordnung zum Ort des vorbestimmten Bereichs sowie der Größe und der Helligkeit des jeweiligen Musters bzw. Marke speichert.

5. Vorrichtung nach Anspruch 4,
dadurch **gekennzeichnet**, daß die Steuereinheit den Helligkeitswert des Lichtstrahls zur Erzeugung von Marken in mehr als zwei Stufen oder kontinuierlich einstellt.

6. Vorrichtung nach einem der Ansprüche 1 bis 5,
dadurch **gekennzeichnet**, daß die Steuereinheit Augenbewegungen kompensiert.

7. Vorrichtung nach einem der Ansprüche 1 bis 6,
dadurch **gekennzeichnet**, daß die Steuereinheit den Beleuchtungslichtstrahl innerhalb der Zeitspanne, in der der Beleuchtungslichtstrahl einen Abtastpunkt beleuchtet, wenigstens einmal abschaltet, d.h. daß der zweite Intensitätswert Null ist.

8. Vorrichtung nach einem der Ansprüche 1 bis 7,
dadurch **gekennzeichnet**, daß die Steuereinheit die Intensität des Beleuchtungslichstrahls zusätzlich zum Umschalten des Intensitätswertes zwischen zwei Intensitätswerten variiert.

9. Vorrichtung nach einem der Ansprüche 1 bis 8,
dadurch **gekennzeichnet**, daß die Steuereinheit einen akusto-optischen Modulator aufweist, der im Lichtweg des Beleuchtungslichtstrahls angeordnet ist.

10. Vorrichtung nach einem der Ansprüche 1 bis 9,
dadurch **gekennzeichnet**, daß die Steuereinheit die Beleuchtungslichtquelle zwischen zwei Intensitätswerten umschaltet bzw. an- und abschaltet.

11. Vorrichtung nach einem der Ansprüche 1 bis 10,
dadurch **gekennzeichnet**, daß eine weitere Lichtquelle vorgesehen ist, die eine Untergrundhelligkeit erzeugt.

12. Vorrichtung nach einem der Ansprüche 1 bis 11,
dadurch **gekennzeichnet**, daß zur Beobachtung des Augenhintergrunds die Beleuchtungslichtquelle Licht anderer Wellenlänge als zur Projektion der Muster emittiert.

13. Vorrichtung nach einem der Ansprüche 1 bis 12,
dadurch **gekennzeichnet**, daß die Steuereinheit einen Speicher aufweist, in dem die Position, Form und Helligkeit der Marken gespeichert sind.

14. Vorrichtung nach Anspruch 13,
dadurch **gekennzeichnet**, daß die Steuereinheit Daten betreffend die Marken bzw. Muster in Zuordnung zum aufgenommenen Bild bzw. markanter Fundusstellen gespeichert enthält.

15. Vorrichtung nach Anspruch 14,
dadurch **gekennzeichnet**, daß die Steuereinheit bei Augenbewegungen eine Korrektur des Ortes der aufleuchtenden Marke und/oder der Zuordnung der soeben aufgeleuchteten Marke zum Augenhintergrund durchführt.

16. Vorrichtung nach einem der Ansprüche 1 bis 15,
dadurch **gekennzeichnet**, daß die Steuereinheit Werte betreffend die Helligkeit der Marken als digitaler Wert speichert, und daß die Zuordnung einer Helligkeit zu einem digitalen Wert in der Steuereinheit nicht linear ist.

17. Vorrichtung nach Anspruch 16,
dadurch **gekennzeichnet**, daß die Steuereinheit einen Digital/Analog-Wandler aufweist, der den digitalen Wert in einen Analogwert wandelt, und dem wenigstens ein nichtlinearer Verstärker nachgeschaltet ist, dessen Ausgangssignal den Helligkeitswert angibt.

## Claims

1. Device for examining the visual field, with
(a) an illuminating light source (1) which generates an illuminating light ray (3),
(b) a detector unit (12) with subsequent evaluation and synchronising unit,
(c) a scanning unit which comprises ray-deflecting elements (5, 8) and ray-imaging elements (6, 7,10) through which the illuminating light ray is imaged along a light path on a pre-set area of the fundus of an eye to be examined (11) which passes the light (4) reflected from the fundus of the eye to the detector unit, from whose temporally sequential output signal the evaluation and synchronising unit generates a point-by-point image of the illuminated area of the fundus of the eye, and
(d) a control unit which controls the intensity of the illuminating light ray (3) scanning the fundus of the eye in such a way that marks or patterns are projected on to a presettable area of the fundus of the eye with a pre programmable brightness which the person being examined perceives or in the event of visual field defects does not perceive,
characterised in that the control unit is designed in such a way that within the time period in which the illuminating light ray illuminates a preset area on to which the marks or patterns are to be projected, it switches the illuminating light ray (3) at least once for a certain fraction of this time period from one first intensity value to at least one second intensity value to set a certain brightness value of the marks or patterns.

2. Device according to Claim 1,
characterised in that the control unit records movements of the eye for an objective visual acuity test and correlates these with the location of the pre-set area on the fundus of the eye on to which the mark(s) or pattern(s) are projected.

3. Device according to Claim 1 or 2,
characterised by the fad that the control unit adjustably changes the pattern brightness and/or size as well as its movement speed as required.

4. Device according to Claim 3,
characterised in that the control unit stores the response "seen" or "not seen" of the person being examined relative to the location of the preset area and to the size and brightness of the corresponding pattern or mark.

5. Device according to Claim 4,
characterised in that the control unit adjusts the brightness of the light ray to generate marks in more than two steps or continuously.

6. Device according to any of Claims 1 to 5,
characterised in that the control unit compensates eye movements.

7. Device according to any of Claims 1 to 6,
characterised in that the control unit switches off the illuminating light ray at least once, i.e. the second intensity value is zero, within the time period in which the illuminating light ray illuminates a scanning spot.

8. Device according to any of claims 1 to 7,
characterised in that the control unit varies the intensity of the illuminating light ray in addition to the switching of the intensity value between two intensity values.

9. Device according to any of Claims 1 to 8,
characterised in that the control unit comprises an acousto-optical modulator which is positioned in the light path of the illuminating light ray.

10. Device according to any of claims 1 to 9,
characterised in that the control unit switches the illuminating light source between two intensity values or switches it on and off.

11. Device according to any of Claims 1 to 10,
characterised in that a further light source is provided which generates a background brightness.

12. Device according to any of claims 1 to 11,
characterised in that the illuminating light source emits light of a different wavelength than for the projection of the patterns to observe the fundus of the eye.

13. Device according to any of Claims 1 to 12,
characterised in that the control unit comprises a memory in which the position, shape and brightness of the marks are stored.

14. Device according to Claim 13,
characterised in that the control unit contains stored data regarding the marks and/or patterns in relation to the image taken and/or significant fundus positions.

15. Device according to Claim 14,
characterised in that in the event of eye movements the control unit performs a correction of the location of the mark lighting up and/or the relation of the mark lighting up to the fundus of the eye at this moment.

16. Device according to any of Claims 1 to 15,
characterised in that the control unit stores values regarding the brightness of the marks as digital values and that the allocation of a brightness to a digital value in the control unit is not linear.

17. Device according to Claim 16,
characterised in that the control unit comprises a digital/analog converter which converts the digital value into an analog value and which is followed in series by at least one non-linear intensifier whose output signal gives the brightness value.

## Revendications

1. Dispositif d'examen du champ visuel comportant
(a) une source lumineuse d'éclairage (1) qui produit un rayon lumineux d'éclairage (3),
(b) un dispositif de détection (12) comportant une unité d'exploitation et de synchronisation montée en aval,
c) un dispositif de balayage qui présente des éléments de déviation (5, 8) des rayons et des éléments de focalisation (6, 7, 10) des rayons, par l'intermédiaire desquels le rayon lumineux d'éclairage est focalisé le long d'un chemin lumineux sur une zone déterminée à l'avance du fond de l'oeil d'un oeil (11) qu'il s'agit d'examiner et qui guident la lumière réfléchie (4) par le fond de l'oeil jusqu'au dispositif de détection à partir du signal de sortie, séquentiel dans le temps, duquel l'unité d'exploitation et de synchronisation produit par points une image de la zone éclairée du fond de l'oeil, et
(d) une unité de commande, qui commande l'intensité du rayon lumineux d'éclairage (3) qui balaie le fond de l'oeil, de façon telle que des marques, ou motifs, sont projeté(e)s sur une zone du fond de l'oeil susceptible d'être déterminée à l'avance, avec une luminosité susceptible d'être fixée à l'avance, que la personne examinée perçoit, ou dans le cas de défauts du champ visuel, ne perçoit pas,
caractérisé en ce que l'unité de commande est agencée de façon telle qu'à l'intérieur d'un intervalle de temps, dans lequel le rayon lumineux d'éclairage éclaire une zone déterminée à l'avance sur laquelle des marques ou motifs doivent être projeté(e)s, cette unité commute le rayon lumineux d'éclairage (3) pour régler une valeur de luminosité déterminée des marques ou motifs au moins une fois pendant une fraction de cet intervalle de temps, d'une première valeur d'intensité à au moins une deuxième valeur d'intensité.

2. Dispositif selon la revendication 1, caractérisé en ce que l'unité de commande capte des mouvements de l'oeil en vue de vérifier objectivement la vision et établit une corrélation avec l'emplacement de la zone déterminée à l'avance sur le fond de l'oeil, sur laquelle la ou les marques, ou le ou les motifs sont projeté(e)s.

3. Dispositif selon la revendication 1 ou 2, caractérisé en ce que l'unité de commande modifie de façon réglable la luminosité et/ou la dimension des motifs ainsi que le cas échéant leur vitesse de déplacement.

4. Dispositif selon la revendication 3, caractérisé en ce que l'unité de commande enregistre la réponse "vu" ou "pas vu" de la personne examinée en relation avec l'emplacement de la zone prédéterminée ainsi qu'avec la dimension et la luminosité du motif, ou de la marque, considéré(e).

5. Dispositif selon la revendication 4, caractérisé en ce que l'unité de commande règle en plus de deux gradations ou en continu la luminosité du rayon lumineux destiné à produire des motifs.

6. Dispositif selon l'une des revendications 1 à 5, caractérisé en ce que l'unité de commande compense les déplacements de l'oeil.

7. Dispositif selon l'une des revendications 1 à 6, caractérisé en ce que l'unité de commande interrompt le rayon lumineux d'éclairage au moins une fois dans l'intervalle de temps pendant lequel le rayon lumineux d'éclairage éclaire un point de balayage, c'est-à-dire que la deuxième valeur d'intensité est nulle.

8. Dispositif selon l'une des revendications 1 à 7, caractérisé en ce que l'unité de commande fait varier l'intensité du rayon lumineux d'éclairage en plus de la commutation de la valeur d'intensité entre deux valeurs d'intensité.

9. Dispositif selon l'une des revendications 1 à 8, caractérisé en ce que l'unité de commande présente un modulateur opto-acoustique, qui est disposé dans le chemin lumineux du rayon lumineux d'éclairage.

10. Dispositif selon l'une des revendications 1 à 9, caractérisé en ce que l'unité de commande commute ou met en marche et à l'arrêt la source lumineuse entre deux valeurs d'intensité.

11. Dispositif selon l'une des revendications 1 à 10, caractérisé en ce qu'une autre source lumineuse est prévue, laquelle produit une luminosité de base.

12. Dispositif selon l'une des revendications 1 à 11, caractérisé en ce que, pour l'observation du fond de l'oeil, la source lumineuse d'éclairage émet de la lumière d'une autre longueur d'onde que celle utilisée pour la projection des motifs.

13. Dispositif selon l'une des revendications 1 à 12, caractérisé en ce que l'unité de commande comporte une mémoire, dans laquelle sont enregistrées la position, la forme et la luminosité des marques.

14. Dispositif selon la revendication 13, caractérisé en ce que l'unité de commande contient en mémoire des données concernant les marques ou motifs, en relation avec l'image photographiée, ou des emplacements marquants du fond de l'oeil.

15. Dispositif selon la revendication 14, caractérisé en ce que l'unité de commande exécute, en cas de mouvements de l'oeil, une correction de l'emplacement de la marque éclairée et/ou l'affectation de la marque effectivement éclairée par rapport au fond de l'oeil.

16. Dispositif selon l'une des revendications 1 à 15, caractérisé en ce que l'unité de commande enregistre des valeurs concernant la luminosité des marques sous forme de valeurs numériques, et en ce que la relation entre une luminosité et une valeur numérique de l'unité de commande n'est pas linéaire.

17. Dispositif selon la revendication 16, caractérisé en ce que l'unité de commande présente un convertisseur numérique/analogique, qui transforme une valeur numérique en valeur analogique et en aval duquel est monté au moins un amplificateur non linéaire, dont le signal de sortie indique la valeur ou l'indice de luminosité.
